(19) 

Europäisches
Patentamt
European
Patent Office
Office européen
des brevets

(11) **EP 1 959 819 B1**

(12) **EUROPEAN PATENT SPECIFICATION**

(45) Date of publication and mention
of the grant of the patent:
**17.03.2010 Bulletin 2010/11**

(51) Int Cl.:
**A61B 5/00** (2006.01)

(21) Application number: **06808648.7**

(86) International application number:
**PCT/GB2006/004371**

(22) Date of filing: **23.11.2006**

(87) International publication number:
**WO 2007/060428 (31.05.2007 Gazette 2007/22)**

(54) **SYSTEM&METHOD FOR ESTIMATING SUBSTANCE CONCENTRATIONS IN BODILY FLUIDS**

SYSTEM & VERFAHREN ZUM SCHÄTZEN VON SUBSTANZ-KONZENTRATIONEN IN
KÖRPERFLÜSSIGKEITEN

SYSTEME ET METHODE POUR ESTIMER DES CONCENTRATIONS DE SUBSTANCE DANS DES
FLUIDES CORPORELS

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **23.11.2005 GB 0523832**

(43) Date of publication of application:
**27.08.2008 Bulletin 2008/35**

(73) Proprietor: **City University
London EC1V OHB (GB)**

(72) Inventors:
• **KYRIACOU, Panayiotis, Anastasios
London EC1V 0HB (GB)**

• **RYBYNOK, Victor, Olagovich
London, EC1V 0HB (GB)**

(74) Representative: **Milhench, Mark Lorne
First Thought IP
35 New Broad Street House
New Broad Street
London EC2M 1NH (GB)**

(56) References cited:
**EP-A2- 0 404 562      WO-A1-00/22413
US-A- 5 370 114      US-A1- 2003 109 998**

## Description

Field of the Invention

This invention relates to systems and methods for estimating substance concentrations in bodily fluids. Particularly preferred embodiments of the present invention relate to systems and methods whereby substance concentrations in blood can be optically estimated non-invasively and *in vivo.*

The teachings of the present invention will be described hereafter with particular emphasis on the estimation of substance concentrations in blood, in particular to glucose concentrations. However, it will be immediately apparent to persons of ordinary skill in the art that the teachings of the present invention may be applied to the estimation of substance concentrations in a variety of different bodily fluids, and as such the following description should not be interpreted as being limited solely to the estimation of substance concentrations in blood. It is also the case that whilst it is preferred for the estimation of substance concentrations to be accomplished in vivo, this is not essential and the system and methods disclosed may equally well be employed for in vitro estimation of substance concentrations in bodily fluids.

Background to the Invention

Blood contains a variety of different substances, some of which are linked to medical conditions. For such substances, if a patient is to be treated correctly it is important for the concentration of substances in the blood to be estimated accurately. Such substances include, for example, glucose, cholesterol, urea, bilirubin, oxygen and others. In many situations, it is important to monitor the patient's condition on an ongoing basis and adjust the treatment to be administered accordingly, and as such it is typical for the concentration of a particular substance to be measured repeatedly.

One illustrative medical condition that requires the concentration of a substance in a patient's blood to be measured frequently is diabetes. Diabetes mellitus interferes with the body's processes for metabolizing glucose, leading to absent, undelivered or insufficient blood insulin. Stores of glucose build in the bloodstream, often causing vascular disease, kidney disease, hypertension, and eyesight degradation, and by reducing blood glucose fluctuations, diabetics (especially type II) can reduce complications.

In type I (juvenile onset) diabetes, the pancreatic cells that produce insulin are treated like foreign bodies and killed, which robs the body of its only insulin source. Researchers believe that these cells share a surface receptor similar to a virus. When the immune system attacks this virus, it mistakenly attacks the insulin-producing cells. Type I diabetes accounts for 10 percent of all diabetes cases and requires intensive glucose monitoring accompanied by insulin injection.

In type II (adult onset) diabetes, either insulin-intolerant cells mysteriously emerge or the pancreas does not make enough insulin. Diet, exercise, and oral medication can help control this less severe form of diabetes, but frequent glucose monitoring remains vital for limiting the glucose fluctuations which instigate diabetic complications.

Monitoring of blood glucose levels can be accomplished by a variety of techniques, some of which are invasive, and others of which are semi-noninvasive or wholly non-invasive.

One commonplace way of invasively measuring a particular substance concentration is to take a sample of the patient's blood and to test that sample for glucose concentration. However, the relatively high frequency with which blood glucose levels need to be monitored in a patient with diabetes means that repeated invasive sampling of blood can cause unnecessary pain, inconvenience and discomfort to the patient. It is also the case that the cost of the equipment (such as lancets and testing strips) associated with this invasive testing process is not insignificant, and this "financial penalty" for frequent testing can discourage patients from rigorously following a prescribed testing timetable.

As an alternative to invasive testing, several indirect semi-noninvasive blood substance measurement techniques have previously been proposed. Some of these techniques still require transcutaneous penetration (i.e. penetration through upper skin layers) to obtain samples of interstitial fluids, and other approaches measure substance concentration in easy accessible body fluids (like saliva or tears). In either case, the concentration of the substance in these interstitial or accessible fluids is then correlated with the substance concentration in blood, but accuracy of the measurement may suffer as it is not unusual for there to be a time delay between changes in the concentration of the substance in the interstitial or easily accessible fluid and changes in the concentration of the substance concentration in the blood. This is especially true of blood glucose levels, where techniques using indirect measurements may allow dangerous levels of blood glucose concentration to either go undetected or to be detected later than is necessary for administering the required treatment to the patient.

A number of other blood substance measurement techniques which have been proposed are classified as being non-invasive, even they though may require the injection of a chemical agent or the administration of a significant (physiologically detectable) amount of electrical energy to the patient. These techniques tend to be relatively inaccurate and often require personal re-calibration which typically involves taking a blood sample from the patient by a traditional invasive technique, thereby offsetting any advantages afforded to the patient by the non-invasive part of the technique.

Many independent researchers have shown that a correlation exists between blood glucose and blood absorbance coefficients of optical radiation (visible and near infrared (NIR)), and similar correlations have been found for other blood substances. Whilst such correlations would tend to suggest that non-invasive optical monitoring may be possible, it is the case that the information that can be obtained by optical sensors through the skin is greatly affected across the whole spectra by other components present in blood and tissue. For example, the optical information that can be obtained for glucose is greatly affected by heavy absorption at similar wavelengths by water and fat. Thus, whilst an experimental optical monitoring system may work adequately when sampling a glass of glucose and water, practical attempts to implement such an approach have proved to be technically problematic, principally because it is difficult to use information obtained from optical sensors to directly predict blood glucose concentration. Attempts to alleviate such problems have been proposed, but such attempts typically involve an additional non-optical experiment to obtain auxiliary information that can help to increase the accuracy of measurements made. Prior art document WO0/22413 discloses such a method.

An important application for blood glucose monitors is in closed loop systems for diabetes management. Closed loop systems can significantly decrease the risk of hypoglycemia (lack of glucose) and long term diabetes complications, but such systems are only achievable with precise and continuous blood glucose monitoring, the like of which is currently unavailable using existing techniques. Clearly it would be advantageous if a system could be devised that integrated a non-invasive monitoring device with a non-invasive drug delivery methods to create a device that may be operated to easily and discretely control diabetes.

In general terms, previously proposed techniques for measuring blood glucose levels which are invasive or semi-invasive can tend to be painful to the patient, particularly given the high frequency with which such tests must be repeated. Techniques that involve measuring blood glucose levels indirectly via interstitial fluid tend to be relatively inaccurate given the time delay between changes in blood glucose levels and changes in glucose concentration in interstitial fluid, and many techniques require personal calibration for each particular patient - which calibration typically requires a sample of the patient's blood to be taken via traditional invasive techniques. Non-invasive techniques involving directing light at a patient's skin have previously been theoretically proposed, but in practice tissue components and other substances blur the optical glucose fingerprint and attenuate the detectable signal.

It is apparent, given the foregoing, that there is a need in the art for a system and method for effectively and non-invasively estimating the concentration of a substance in blood, which method and system are not hampered by requiring personal calibration.

Statement of the Invention

To this end, a presently preferred embodiment of the present invention provides a method for estimating the optical response of a subject comprising blood, the method comprising forming a model whereby the spectral response of a subject to incident light can be estimated, the model including a plurality of parameters, each parameter corresponding to the influence on the spectral response of a respective biochemical component, directing light of a known spectral content at the subject, detecting the spectral response of the subject to the light and repeatedly performing the steps of: i. modelling the spectral response of the subject to the light by means of the model; ii. comparing the modelled spectral response with the detected response; and iii. altering the model in dependence on that comparison by varying one or more of the said parameters.

Preferably the step of altering the model is only performed if the modelled spectral response is closer to the detected response than the modelled spectral response of the current model.

Light comprising multiple wavelengths is preferably directed at the subject and the spectral response of the subject is detected for each respective wavelength. The light directed at the subject may be generated by multiple devices, each being capable of generating light consisting of a single respective wavelength.

The number of multiple wavelengths is preferably greater than or equal to the number of parameters included in the model.

Preferably the steps of modelling, comparing and altering are performed repeatedly until a difference between the modelled spectral response and the detected response is below a predetermined threshold.

The step of altering the model may be performed using a neural network.

The spectral response of the subject may be detected in light passing through two different paths through the subject.

Preferably the model includes parameters representing the light scattering and light absorption properties of the biochemical components. The parameters may include the concentrations of one or more of fat, water and glucose in the subject and the multiple wavelengths may include $2.10\mu m$, $2.27\mu m$ and $2.32\mu m$.

According to a second aspect of the invention, there is provided a method for manufacturing a non-invasive sensor for estimating the concentration of a biochemical component in a subject comprising blood, the method comprising forming an altered model by the method disclosed above, forming a processing device configured to apply the model so as to estimate, in dependence on the spectral response of a subject to light of a known spectral content, the influence on the spectral response of the biochemical component and providing output means whereby the processing device can cause data generated in dependence on the estimated influence to be output to a user.

According to a third aspect of the invention, there is provided a method for estimating the concentration of a biochemical component in a subject comprising blood, the method comprising forming an altered model by the method disclosed herein, directing light of a known spectral content at the subject, detecting the spectral response of the subject to the light and applying the model so as to estimate, in dependence on the spectral response of the subject to the light, the influence on the spectral response of the biochemical component and in dependence on the estimated influence, estimating the concentration of the biochemical component in the subject.

A fourth presently preferred embodiment of the present invention relates to a system for estimating substance concentrations in bodily fluids, the system comprising: means for illuminating a subject comprising bodily fluid with light having a known spectral content; means for detecting the subject's spectral response to said light; and processing means operable to establish a model of the subject's spectral response to irradiation with light of said known spectral content, the model having a plurality of parameters each of which corresponds to a respective biochemical substance's influence on the spectral response; wherein said processor is further operable to compare the modelled spectral response with the detected spectral response, and in the event that said modelled spectral response differs from said detected spectral response by more than a predetermined amount to iteratively adjust one or more of said parameters until said difference is less than said predetermined amount.

In a preferred embodiment the processor is further operable, following an adjustment which yields a difference that is less than said predetermined amount, to derive from said model an estimation of the concentration of a said biochemical component in said subject.

Another aspect of the invention relates to a probe for use with the system described above, the probe comprising a plurality of optical emitters and one or more photodetectors arranged side-by-side in a single housing, the arrangement being such that when the probe is placed adjacent to a subject, the plurality of optical emitters are operable to illuminate the subject with light of a known spectral content, a least a portion of which travels through the subject for detection by one or more of said photodetectors.

A fifth presently preferred embodiment of the present invention relates to a computer program comprising one or more computer program elements that are operable, when executed in an execution environment, to form a model whereby the spectral response of a subject to incident light can be estimated, the model including a plurality of parameters, each parameter corresponding to the influence on the spectral response of a respective biochemical substance.

Other presently preferred embodiments and advantages of the present invention will be apparent to persons of ordinary skill in the art following a consideration of the following detailed description of preferred embodiments.

Brief Description of the Drawings

Presently preferred embodiments of the present invention will now be described, only by way of illustrative example, with reference to the accompanying drawings, in which:

Fig. 1 illustrates the principle functional and physical components for the development of a tissue model;
Fig. 2 illustrates an optical sensor configured to implement the model derived by the arrangement of Fig. 1;
Fig. 3 illustrates an adaptive modelling scheme for measuring blood glucose - concentration;
Fig. 4 illustrates an optical emitter and detector;
Fig. 5 is a schematic representation of a novel sensor probe;
Fig. 6 is a schematic representation of a system according to a preferred embodiment of the present invention;
Fig. 7 is a schematic representation of optical driver circuitry;
Fig. 8 is a schematic representation of detector driver circuitry; and
Fig. 9 is a flowchart depicting an illustrative mode of operation for the system disclosed in Fig. 6.

Detailed Description of Preferred Embodiments

Before describing preferred embodiments of the present invention in detail, it s worthwhile reiterating at this juncture that whilst the teachings of the present invention are described hereafter with particular emphasis on the estimation of substance concentrations in blood (in particular to glucose concentrations), the teachings of the present invention may be applied to the estimation of substance concentrations in a variety of different bodily fluids, and as such the following description should not be interpreted as being limited solely to the estimation of substance concentrations in blood. It is also the case that whilst it is preferred for the estimation of substance concentrations to be accomplished in vivo, this is not essential and the system and methods disclosed may equally well be employed for in vitro estimation of substance concentrations in bodily fluids.

As will hereafter be apparent, embodiments of the present invention provide techniques for estimating the concentration of a substance in blood by directing light at a subject and measuring the spectral response of the subject to that light In general terms, the techniques proposed involve adaptively modelling the subject's spectral response to incident light and comparing that model to a measured response to incident light. The model includes parameters representing the readily estimatable contribution that biochemical substances such as water, fat or haemoglobin make to the spectral response of the subject as well as a parameter representing the unknown contribution made to the spectral response by the substance of interest, for example glucose. The model is adapted until a spectral response estimated by the model is within an acceptable error margin of a detected spectral response for a given intensity of incident light. The adapted model forms the core of a system for estimating the concentration of the substance in a patient's blood. In general terms, the system directs light at the subject, which may be a patient's finger, ear lobe or other body part, detects the spectral response of the subject and uses the model and the detected response to calculate the concentration of the substance in the patient's blood.

The system includes an optical sensor and is capable of separating out the contribution that each of the biochemical substances in the subject makes to the spectral response of the subject. This enables the sensor to determine the contribution made by one specific component, thereby enabling the concentration of that substance in the subject to be calculated. The sensor is thus able to accurately and non-invasively calculate an estimated concentration of a substance in the subject, without requiring an invasive calibration process.

The spectral response of a part of the human body to incident light is dependent on the biochemical substances it contains. At specific wavelengths of incident light, the spectral response of the body part is dependent on the absorption and scattering properties of each substance at that wavelength. In particular, the concentration of each substance and its associated location within the subject (i.e. the extent to which the component is located in the transmission path of the light directed at the subject) contribute to a greater or lesser extent to the resulting spectrum.

For example, if the relevant substances are X, Y and Z, then the spectral response of the body part to incident light is some function of X, Y and Z at each wavelength. The function is determined by directing light at a subject and repeatedly adapting a model until the model outputs an estimated light intensity that is within an acceptable error margin of a detected light intensity. The resulting model is then representative of the relationship between detected light intensity and the concentrations of substances X, Y and Z for specific wavelengths of incident light.

If substance X is the substance whose concentration it is desired to estimate, then light including the specific wavelengths on which the model is based (i.e. light of wavelengths that X, Y and Z are responsive to) is directed at the subject and the detected light intensity at each wavelength is fed into the model. The output of the model is compared to the measured light intensity and the parameters defining the model are altered until the modelled light intensity is within an acceptable margin of the detected light intensity. At this point the model is considered to be representative of the subject, and the concentration of substance X in the subject can then be calculated from the model.

If altering the model parameters causes the difference between the estimated spectral response and the actual spectral response to increase, the adaptive algorithm recognises that the alterations have not resulted in a more accurate model. The algorithm may then continue with the current model, i.e. the model before it was altered, and adapt its parameters in a different way to try and decrease the difference between the estimated spectral response and the detected spectral response.

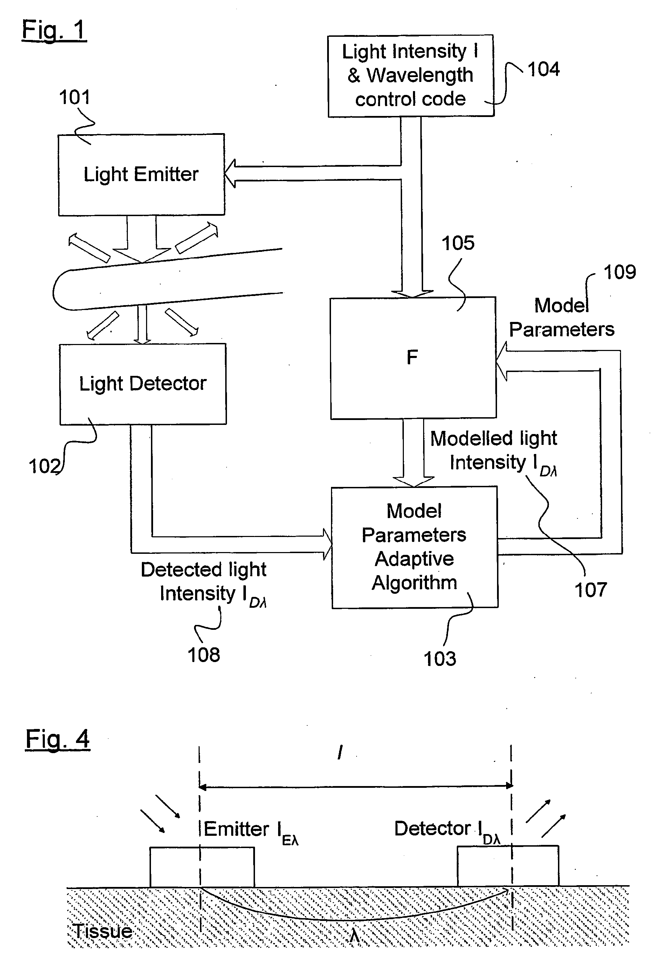

The functional steps of the model adaptation technique are illustrated schematically in Fig. 1. As shown, a light emitter 101 is arranged to direct light at a subject which may comprise a part of a patient's body or a suitable body simulation, such as a tissue phantom comprising anatomical components (e.g. skin, fat, blood) or simulations thereof. In Fig. 1 the subject comprises a patient's finger, but other body parts such as the ear lobe may instead be investigated. Fingers tend to be relatively highly vascularised and are therefore a convenient site for performing this technique. However, it is important to note that any suitable part of the body may be used. For example, the ear lobe has been proposed as a particularly suitable site given the relatively low amounts of other materials that may shield the optical response attributable to glucose.

The reflected or transmitted light is detected by a light detector 102 and the resulting output is fed into an adaptive model parameters algorithm 103. The algorithm also receives a modelled light intensity 107 that is generated by a modelling unit 105 which is configured to model the absorption and scattering characteristics of the tissue on which the light is incident. This model is used to estimate the intensity of the light transmitted and/or reflected through the tissue for a given emitted light intensity 104.

The adaptive model parameter algorithm 103 is configured to compare the light intensity estimated by the model with the detected light intensity 108 and to adjust model parameters 109 iteratively until a difference between the estimated intensity and the detected intensity is below a predetermined threshold. When the difference between the estimated and detected intensities is within this acceptable margin of error, the model is considered to be an acceptable model of the spectral response of the subject.

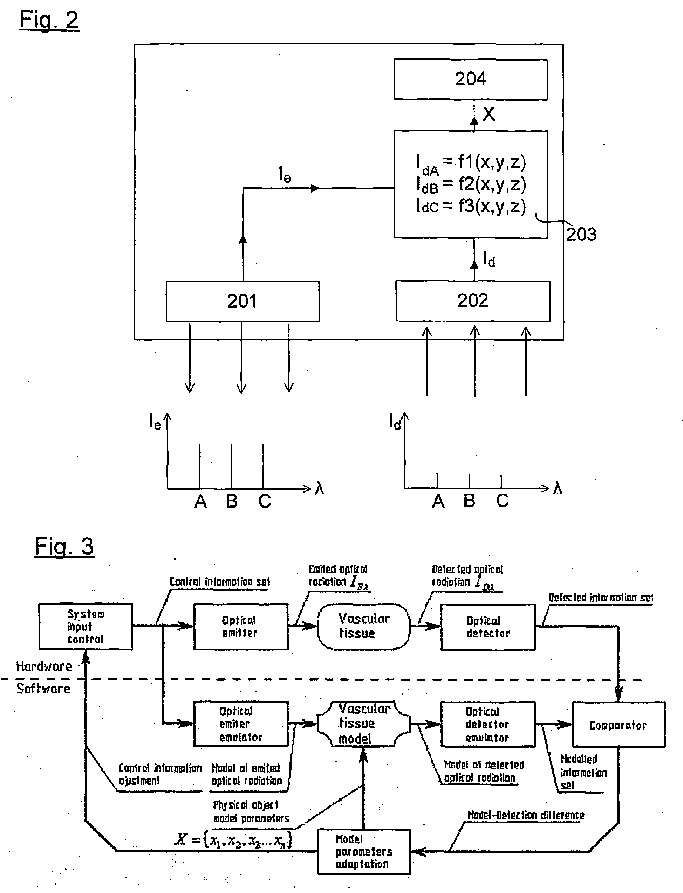

Once the altered model has been derived, it may then be employed to derive an estimation of substance concentration in the subject, and the functional steps of this process are depicted schematically in Fig. 2.

As shown, a light emitter 201 is configured to direct light at a subject, for example a patient's body part, and a light detector 202 is configured to detect light reflected from and/or transmitted through the subject. A concentration calculating unit 203 is provided and is operable to calculate from the altered model, in a manner hereafter described, the concentration of a substance under investigation in the subject and to provide that concentration to a user interface 204 (such as a display).

Considering the aforementioned example of relevant biochemical substances X, Y and Z, the concentration calculating unit is configured to implement a model that includes three functions respectively defining the spectral response of the subject due to substances X, Y and Z to light of known intensity and wavelengths A, B and C. The light detector measures the intensity of light transmitted through and/or reflected from the subject and passes this information to the concentration calculating unit, which calculates the concentration of substance X using the model in a manner that is hereafter described. This value is then passed to the user interface which outputs it to the user.

The chosen wavelengths A, B and C are preferably wavelengths at which substance X displays a strong spectral response, e.g. wavelengths at which it absorbs heavily. The number of wavelengths at which the spectral response of the subject is measured should be at least equal to the number of unknowns in the model. In this case, the concentration of biochemical substances X, Y and Z are unknown, so the model represents the spectral response of the subject at three different wavelengths: A, B and C.

The light emitter is preferably capable of emitting a spectrum of light containing specific wavelengths. This may be achieved by using a range of single emitters, each of which is capable of emitting light at a single specific wavelength. For example, a bank of light emitting diodes (LEDs) that emit a light of a single wavelength may conveniently be used. It will be appreciated, however, that a variety of different emitters may instead be employed. For example, the emitters may comprise optic fibers arranged to emit light of different wavelengths.

The adaptive modelling technique will now be described in more detail with specific reference to the estimation of a blood glucose concentration. As mentioned above, this application is purely illustrative and the teachings of the present invention may be used for measuring the concentration of any suitable substance in the blood of a patient. For example, embodiments of the invention could also be used to measure concentration of other blood analytes, such as e.g. urea, bilirubin and oxygen.

The adaptive modelling technique described herein is different from previously proposed adaptive techniques in the sense that no additional non-optical experiments are used. Instead, the amount of information obtained by the optical sensors and known from human physiology and biochemistry may be systematically increased, and an algorithmic scheme is provided to analyse this information. This adaptive modelling technique has an important advantage: it allows measurement accuracy to be gradually increased by controllably increasing the system complexity, i.e. the amount of information used in the blood glucose prediction model and adaptation algorithm, in particular the complexity of and the number of parameters used in the model.

The adaptive modelling measurement technique herein described is a generic non-empirical analytical method, designed to alleviate calibration problems that have hitherto caused problems for the accurate non-invasive estimation of subject substance concentration, in particular glucose concentration. This technique (the mathematical basis for which will now be described in connection with Figs. 3 & 4) can be implemented using either transmittance or reflectance photometric techniques applied to *"Vascular tissue"* of diabetic patients.

Properties of interest of the *"Vascular tissue"* are represented numerically by a vector of parameters $X = \{x_1, x_2, x_3,...x_n\}$. The emitted optical radiation wavelength and intensity $I_{E\lambda}$ is selected so that alteration of any chosen property of the "*Vascular tissue*" will produce a detectable change in the detected optical radiation $I_{D\lambda}$. One of the properties of the *"Vascular tissue"* is glucose concentration, which is represented by one of the parameters in the vector $X$. The relationship between emitted and detected optical radiation is established by a mathematical approximation of the *"Vascular tissue"*, which is defined in Fig. 3 as the "*Vascular tissue model*". When the parameters in the vector $X$ match, within predefined tolerances, the properties of the *"Vascular tissue"* then the "*Model-detection difference*" will become small and the vector $X$ can be accepted as an acceptable description of the real properties of the vascular tissue.

Mathematically the existence of more than one vector $X$ producing a small "*Model-detection difference*" is possible, and as such it is preferred for the optical emitter to emit radiation with a number of different intensities, wavelengths and in different geometrical positions in respect to the optical detector for a given control information set. For example, the emitter and detector may be arranged so that light that has passed via two (or more) different paths through subject is detected. The detector is then configured so that it detects the light of the two beams that are transmitted and/or reflected by the subject. The correct vector X will be the one which produces the smallest model-detection difference for a sufficiently large number of control information sets.

This technique allows for continuous auto-calibration of the blood glucose measurement system, so that it can be made available to all diabetic patients without the need for individual re-calibration.

In order for the adaptive modelling measurement method to be implemented practically, certain assumptions. These assumptions include physical assumptions (for example how light is absorbed by the subject, and how light propagates through and is scattered by the subject), and assumptions about physiological tissue properties (such as tissue structure and scattering properties of its different parts, and tissue light absorbing components and their distribution throughout the subject).

The model is built by increasing the number of assumptions in a step-by-step fashion. As will now be described the subject model is created by means of a *Signal Separation Information Extraction (SSIE)* method that includes two steps, a first signal separation step and a second information extraction step. The SSIE method hereafter described uses the tissue light absorption assumption illustrated in Fig. 4, and the following symbols used in Fig. 4 and hereafter have the following meanings:

$\lambda$ - radiation wavelength
$I_{E\lambda}$ - emitted radiation intensity, with wavelength $\lambda$
$I_{D\lambda}$ - detected radiation intensity, with wavelength $\lambda$
$l$ - Emitter-Detector distance

*Signal separation*

If $I_{L\lambda}$ is defined as being radiation and intensity losses in the tissue, then for wavelength $\lambda$ :

$$I_{L\lambda} = I_{E\lambda} - I_{D\lambda} \qquad\qquad 1$$

Modern quantum theory of light states that intensity losses occur due to the different kinds of photon absorption and scattering. So, if we define $S_\lambda$ as losses occurring due to the scattering and $A_\lambda$ as losses due to the absorption for radiation of wavelength $\lambda$, then we can write:

$$I_{L\lambda} = S_\lambda + A_\lambda \qquad\qquad 2$$

Suppose we have a model describing behaviour of radiation in tissue, not necessarily explaining the real physical nature of phenomena, but giving a good approximation of what is seen in experimental data ($I_{L\lambda}$). The model should allow the function parameterization of tissue properties and signal separation as defined in equation 2, and can be represented as follows:

$$E_\lambda = F(P_\lambda, l, I_{E\lambda}) + G(H_\lambda, l, I_{E\lambda}) \qquad\qquad 3$$

Where $E_\lambda$ is the model approximation for the radiation intensity losses in tissue $I_{L\lambda}$; $F(P_\lambda, l, I_{E\lambda})$ is a parameterized function approximating for losses occurring due to scattering $S_\lambda$; and $G_\lambda(H_\lambda, l, I_{E\lambda})$ is a parameterized function approximating for losses occurring due to absorption $A_\lambda$. $P_\lambda = \{p_{1\lambda}, p_{2\lambda}, p_{3\lambda} ... p_{n\lambda}\}$ and $H_\lambda = \{h_{1\lambda}, h_{2\lambda}, h_{3\lambda} ... h_{m\lambda}\}$ are parameters describing the scattering and absorption property of tissue.

If we substitute the correct set of parameters $P_\lambda$ and $H_\lambda$ into this model we should get:

$$|I_{L\lambda} - E_\lambda| = \Delta \qquad\qquad 4$$

Where $\Delta$ is the approximation error. This should be less than some pre-required value, which can be defined according to the required precision for the end measurement results. For example, the required precision for blood glucose measurement may be in the region of 10%, preferably less than 10%, more preferably less than or equal to 5%.

If condition 4 is satisfied for a "sufficiently high number" of different sets ($l, I_{E\lambda}, I_{L\lambda}$) obtained practically then we can make good assumption that:

$$F(P_\lambda, l, I_{E\lambda}) \rightarrow S_\lambda \qquad\qquad 5$$

$$G(H_\lambda, l, I_{E\lambda}) \rightarrow A_\lambda \qquad\qquad 6$$

Where the symbol "→" means "tends towards" (as it is not possible to actually measure $S_\lambda$ and $A_\lambda$).

To find the minimal "sufficiently high number" of different sets consider the following example of a simple parametrical equation.

$$z = ax + bx - cy$$

Suppose we know the form of the equation (linear in this case), and any correct set of dependent and independent variables $(x,y,z)$. We need to find the equation parameters $(a,b,c)$. There are three parameters in this equation, so a system of equations with a minimum of three independent equations is required. We can create this system of equations by using three (or more) sets of variables $(x,y,z)$:

$$\begin{cases} z_1 = ax_1 + bx_1 - cy_1 \\ z_2 = ax_2 + bx_2 - cy_2 \\ z_3 = ax_3 + bx_3 - cy_3 \end{cases}$$

If the equations are independent, then we can find the parameters $(a,b,c)$ by solving this system of equations.

Thus, the number of parameters used in equation 3 defines the "sufficiently high number" of different values of $l$, $I_{E\lambda}$ and $I_{L\lambda}$. As the equation parameters for the tissue model are $P_\lambda = \{p_{1\lambda}, p_{2\lambda}, p_{3\lambda}...p_{n\lambda}\}$ and $H_\lambda = \{h_{1\lambda}, h_{2\lambda}, h_{3\lambda}...h_{m\lambda}\}$, the minimum number of different parameter sets $(l, I_{E\lambda}, I_{L\lambda})$ is $m+n$. This number of parameter sets allows us to create system of equations, by solving which we can find all of the parameters $P_\lambda$ and $H_\lambda$, which describe the absorption and scattering properties of the tissue at wavelength $\lambda$. The particular model considered to find the $m+n$ sets of $(l, I_{E\lambda}, I_{L\lambda})$ should not produce any dependency between the equations in the equation system.

Depending on the exact form of the equations, it is not necessarily straightforward to solve the system of equations directly. Thus, an approximating (or step approximating) algorithm may be applied to find $P_\lambda$ and $H_\lambda$. However, it is still necessary to have $m+n$ different sets of parameters $(l, I_{E\lambda}, I_{L\lambda})$ to use as feedback for a successful approximation.

It is desirable for the tissue model to obey the following law: Let $P_{1\lambda} \neq P_{2\lambda}$ and $H_{1\lambda} \neq H_{2\lambda}$ for any $P_{1\lambda}, P_{2\lambda}, H_{1\lambda}, H_{2\lambda}$ then $F(P_{1\lambda}, l, I_{E\lambda}) \neq F(P2_\lambda, l, I_{E\lambda})$ and $G(H_{1\lambda}, l, I_{E\lambda}) \neq G(H_{2\lambda}, l, I_{E\lambda})$ for any $l$ and $I_{E\lambda}$.

Mathematically it is possible for there to be two or more solutions to the system of equations, i.e. two or more $(P_\lambda, H_\lambda)$ sets. The model should be able to determine which of the solutions is physically correct. If the model obeys the above law, then mathematically only one solution is possible and this problem disappears. However, if the model does not obey the above law, another solution is to detect light that has passed through two or more different paths through the object, e.g. by directing two beams of light at the subject, as explained above. Any solution that is not physically correct can be detected and discounted, as it will not estimate the detected intensity correctly for each of the transmission paths.

*Information Extraction*

The Bear-Lambert equation is a well tested empirical law. The main assumption made during its derivation is that for very small parts of material, the radiation energy absorbed by any particular material substance is proportional to the concentration of that substance in the material. We also use this assumption, but with the additional assumption that if there are two or more absorbing substances in the material then the total absorption will be the sum of each separate absorption component for small parts of material. This last assumption is used to create the system of equations 7 shown below.

The tissue is assumed to contain $k$ physical components which absorb radiation on wavelength $\lambda_1$. Any other physical components of the tissue apart from those k components are assumed not to absorb radiation on that wavelength. If there exists a set of wavelengths $\{\lambda_2, \lambda_3, \lambda_4...\lambda_k\}$ where for each physical component $i$ a wavelength $\lambda_i$ exists at which that component absorbs radiation and at which any of the other components from the $k$ may (or may not) also absorb, and if radiation intensity losses $G(H_\lambda, l, I_{E\lambda}) \rightarrow A_\lambda$, then we can write:

$$\begin{cases} A_{\lambda 1} = U_{1\lambda 1} + U_{2\lambda 1} + ... + U_{i\lambda 1} + ... + U_{k\lambda 1} \\ A_{\lambda 2} = U_{1\lambda 2} + U_{2\lambda 2} + ... + U_{i\lambda 2} + ... + U_{k\lambda 2} \\ ... \\ A_{\lambda i} = U_{1\lambda i} + U_{2\lambda i} + ... + U_{i\lambda i} + ... + U_{k\lambda 2} \\ .... \\ A_{\lambda k} = U_{1\lambda k} + U_{2\lambda k} + ... + U_{i\lambda k} + ... + U_{k\lambda k} \end{cases} \qquad 7$$

Where $U_{i\lambda j}$ is part of the radiation intensity loss at wavelength $\lambda_j$ occurring due to absorption by tissue component $i$.

To complete the tissue model we can use the signal separation model defined in equation 2 or use another convenient "tissue phantom like" model.

Using the model defined in equation 2 we can create an equation for each absorbing component i in the tissue, which relates its concentration $c_i$ and absorbed radiation $U_{i\lambda j}$ on wavelength $\lambda_j$ and emitted radiation intensity $I_{E\lambda j}$. To create the complete model we also need the absorption coefficient of each component (or the light absorption spectra of that component) and the scattering property of the tissue, which was found during signal separation:

$$U_{i\lambda j} = L(c_i, Y_{i\lambda j}, P_{\lambda j}, I_{E\lambda j}) \qquad 8$$

Where $P_\lambda = \{p_{1\lambda}, p_{2\lambda}, p_{3\lambda}...p_{n\lambda}\}$ are the parameters describing the scattering property of tissue and $Y_{i\lambda j} = \{y_{i\lambda j,1}, y_{i\lambda j,2}, y_{i\lambda j,3}...y_{i\lambda j,q}\}$ are the parameters describing the absorption property of the each component $i$ on wavelength $\lambda_j$. So, the system of equation 7 can be rewritten in the following form:

$$\begin{cases} G(H_{\lambda 1}, l_1, I_{E\lambda 1}) = L(c_1, Y_{1\lambda 1}, P_{\lambda 1}, I_{E\lambda 1}) + ... + L(c_i, Y_{i\lambda 1}, P_{\lambda 1}, I_{E\lambda 1}) + ... + L(c_k, Y_{k\lambda 1}, P_{\lambda 1}, I_{E\lambda 1}) \\ G(H_{\lambda 2}, l_2, I_{E\lambda 2}) = L(c_1, Y_{1\lambda 2}, P_{\lambda 2}, I_{E\lambda 2}) + ... + L(c_i, Y_{i\lambda 2}, P_{\lambda 2}, I_{E\lambda 2}) + ... + L(c_k, Y_{k\lambda 2}, P_{\lambda 2}, I_{E\lambda 2}) \\ ... \\ G(H_{\lambda i}, l_i, I_{E\lambda i}) = L(c_1, Y_{1\lambda i}, P_{\lambda i}, I_{E\lambda i}) + ... + L(c_i, Y_{i\lambda i}, P_{\lambda i}, I_{E\lambda i}) + ... + L(c_k, Y_{k\lambda i}, P_{\lambda i}, I_{E\lambda i}) \\ .... \\ G(H_{\lambda k}, l_k, I_{E\lambda k}) = L(c_1, Y_{1\lambda k}, P_{\lambda k}, I_{E\lambda k}) + ... + L(c_i, Y_{i\lambda k}, P_{\lambda k}, I_{E\lambda k}) + ... + L(c_k, Y_{k\lambda k}, P_{\lambda k}, I_{E\lambda k}) \end{cases} \qquad 9$$

When a sufficiently accurate form for the model has been determined using the above equations, the only unknown variables in this system of equations are the component concentrations $c_i$. By solving this system of equations, we can find the component concentrations.

In the method described above a static tissue model was considered. However, the optical properties of living tissue can change with time, mainly because of the blood circulation in the short term and because of changes in tissue structure in the long term. As long term changes occur very slowly in comparison with the measurement rate, these changes can be neglected during the time it takes to take a measurement. It is possible that short term changes cannot be neglected, however, and in this case the following strategy can be implemented.

It is assumed that short term changes are periodical in nature, as they are mainly due to the beating of the heart. If so, we can expect that parameter sets describing properties of tissue $P_\lambda$ and $H_\lambda$ will change periodically as well. Let the signal period be $T$ and the parameters obtained at time $t_1$ be $P_{1\lambda}$ and $H_{1\lambda}$. If $P_{2\lambda}$ and $H_{2\lambda}$ are parameters obtained at $t_2 = t_1 + T$ then $P_{1\lambda} = P_{2\lambda}$ and $H_{1\lambda} = H_{2\lambda}$. Or in other words, we can consider the tissue as static for any time $t + nT$, where $n$ is a natural number.

In the preferred embodiment a neural network, adaptive filter, genetic algorithm or other equivalent algorithm may be employed to enable the adaptation of the subject model.

Preferably, the emitter and detector may be arranged so that light that has passed via two different paths through subject is detected. As explained above, this enables any model that is not physically correct to be detected and discounted, as it will not correctly estimate the detected intensity for all transmission paths. In one arrangement, this may be achieved e.g. by arranging the emitter to transmit two beams of light, which are at 90° to each other, through the subject. The detector is then correspondingly configured so that it detects the light of those two beams that is transmitted and/or reflected by the subject. Another arrangement is later described.

One of the biochemical components used in the model is blood glucose concentration. Other components may typically include water and fat, which absorb heavily on the wavelengths at which glucose absorbs. The optical signature of glucose shows that it absorbs most heavily at $2.10\mu m$, $2.27\mu m$ and $2.32\mu m$. Therefore, the detector preferably directs light that includes the wavelengths given above (i.e. $2.10\mu m$, $2.27\mu m$ and $2.32\mu m$) at the patient's skin.

As mentioned above, the technique disclosed herein may be used for measuring the concentration in a patient's blood of substances other than glucose. For example, the technique may be used to generate a model for use in detecting the concentration of other blood analytes, such as e.g. urea, bilirubin and oxygen. The model should incorporate suitable biochemical substances for the substance concentration that is to be estimated, i.e. biochemical substances which show significant absorption at wavelengths at which the substance to be measured absorbs.

The adaptive modelling technique disclosed herein provides many advantages over existing systems, which advantages include the following:

- It does not require insertion of any extrinsic molecules under the skin or into the blood flow.
- There is either no physiological sensation to the patient during measurements, or if there is any sensation, it is very limited.
- It does not require ionization, heating, constriction or any other deformation of human body tissue.
- It is not limited to being used on a particular part of the body.
- It does not require personal calibration.
- The accuracy achievable is limited only by the capability of the optical sensor that is used.

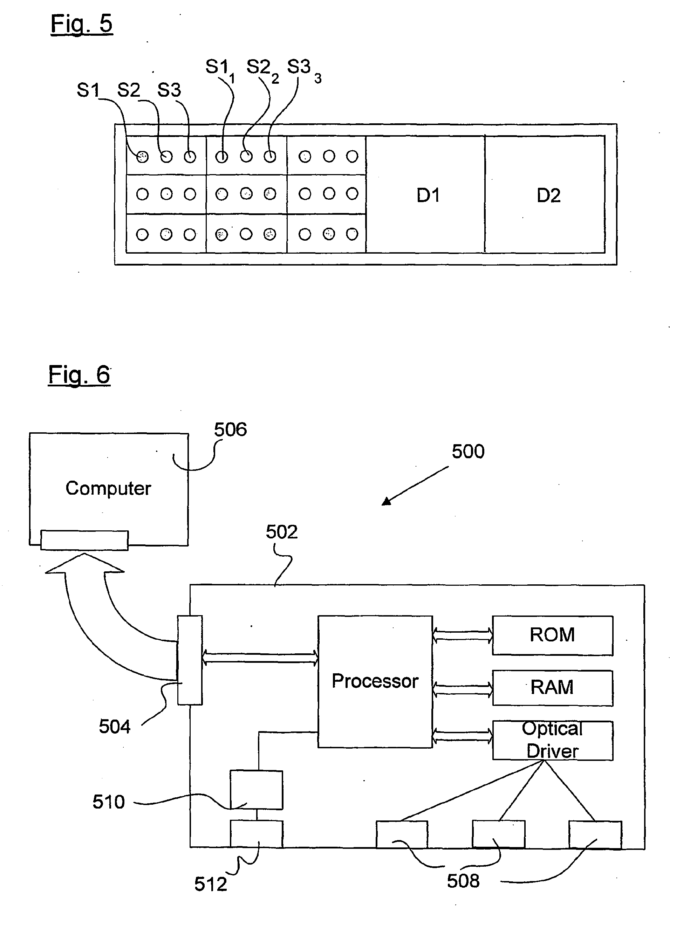

Referring now to Fig. 5, there is shown a schematic view of the underside of a particularly preferred optical probe for use with the system of a preferred embodiment of the present invention.

The probe 400 of this embodiment includes light emitters (S1, S2...etc) that are integrated with photodetectors (D1, D2) into a single housing. The light emitters are arranged in banks of three emitters, with the first emitter (S1, $S1_1$...et) of each bank emitting light at a first wavelength $\lambda_1$, the second emitter (S2, $S2_1$...etc) of each bank emitting light at a second wavelength $\lambda_2$, and the third emitter (S3, $S3_1$...etc) of each bank emitting light at a third wavelength $\lambda_3$. As in the arrangement described above, each of these wavelengths is tailored for the detection of particular substances within the subject. By providing nine banks of emitters and two detectors, it is possible to take measurements with eighteen different spatial relationships between emitter and detector for each wavelength, and in general terms increasing the number of banks of emitters increases the spatial resolution that can be obtained and hence improves the accuracy of the system as a whole. That said, it is possible to operate the system with a fewer number of emitter banks and with only a single detector, but such an arrangement would provide less accurate results than the probe depicted in Fig. 5. As a minimum it is necessary to provide two different paths through the subject for each wavelength. This may be accomplished by providing two emitters for each wavelength and a single detector, or a single emitter for each wavelength and a pair of detectors.

As before the emitters for this probe are preferably LEDs, but it will be apparent that other optical emitters could instead be provided. For example, the emitters could each comprise an optic fibre or bundle of fibres, with light from appropriate sources being injected into the fibres to illuminate the subject at different wavelengths. The probe depicted in fig. 5 is particularly compact, and hence well suited for integration into a small portable estimation system.

Referring now to Fig. 6, there is depicted an estimation system 500 according to a presently preferred embodiment of the present invention. The system of this embodiment comprises an estimation module 502 that includes an I/O interface 504 that enables the module to communicate with a computer 506 that is configured to process data gathered by the estimation module, and to display the results of that processing and other information to a user. In a particularly preferred arrangement the I/O interface is a USB interface, although other equivalent interfaces will be immediately evident to persons of ordinary skill in the art.

The I/O interface 504 is coupled to a processor that is in turn coupled to RAM and ROM memory. Although the memory is shown as being separate from the processor, it will be apparent that the memory may be incorporated with the processor into a microcontroller, and a particularly preferred microcontroller for use in this embodiment is the Atmel AVR family chip ATMEGA8515-16PC. The processor is responsible for driving the optical emitters and detectors, and for gathering data responsive to a scan of the object of interest for output to the computer.

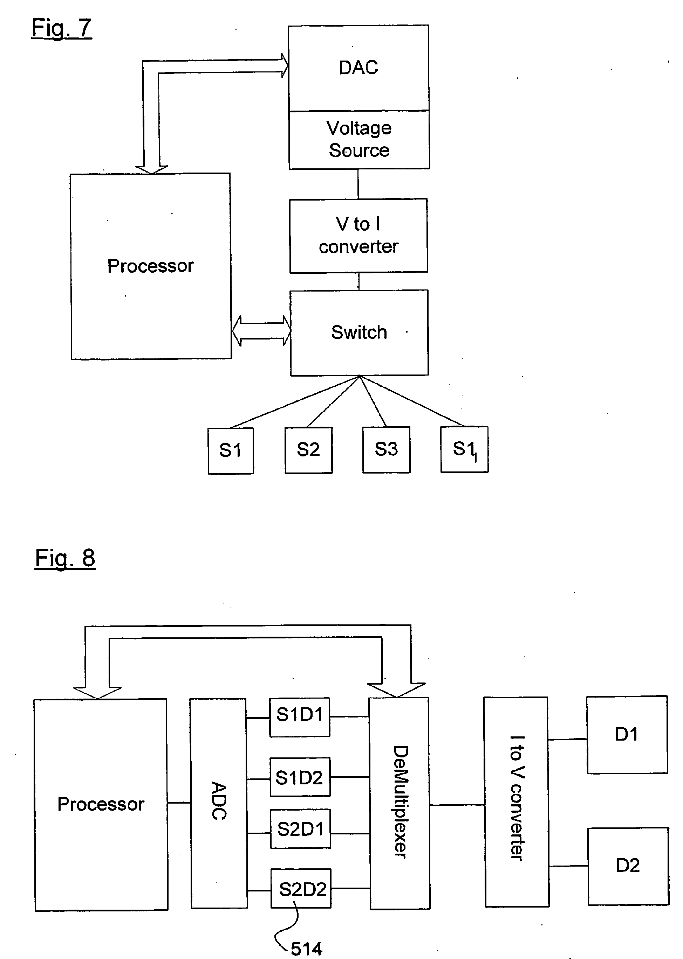

The processor is coupled to optical driver circuitry that is in turn coupled to a plurality of output ports (only three of which are shown) to which the optical emitters are coupled. Further details of the optical drive circuitry will be discussed in connection with Fig. 7 hereafter.

The processor is also coupled to detector driver circuitry 510 that is in turn coupled to a detector port 512 (only one of which is shown) to which a detector is coupled. Further details of the detector drive circuitry will be discussed in connection with Fig. 8 hereafter.

Referring now to Fig. 7, the optical drive circuitry comprises a digital to analogue converter DAC that is coupled to the processor and is configured to receive signals from and transmit signals to the processor. The ADC includes a voltage source and is coupled to a voltage to current converter to provide a current for driving each of the emitters S1, S2 etc. The output from the voltage to current converter is coupled by way of a processor controlled switch to each of the emitters, and the switch is controllable by the processor to energise each of the emitters in turn.

Referring now to Fig. 8, the detector drive circuitry 510 comprises a current to voltage converter that is coupled to (in this instance) each of the detectors D1, D2. The voltage output of the converter is passed by way of a processor controlled multiplexer to a plurality of identical signal conditioning and filtering circuits, and the multiplexer is controllable by the processor to output on each channel thereof a signal representing the intensity measured for a particular emitter detector combination and to switch between channels as different emitter detector pairs are energised. For example, as shown, the first channel may correspond to measurements resulting from illumination of detector D1 by emitter S1, the second channel may correspond to measurements resulting from illumination of detector D2 by emitter S1, and so on. The outputs of each multiplexer channel are passed via an analogue to digital converter to the processor for further processing as herein before described.

Optionally the multiplexer and filtering circuitry may be implemented in software (for executing by the processor of the estimation module or indeed for execution by the processor of the computer), and in this instance the I to V converter would output to a fast ADC, and the signals from the ADC would be further processed by the aforementioned software.

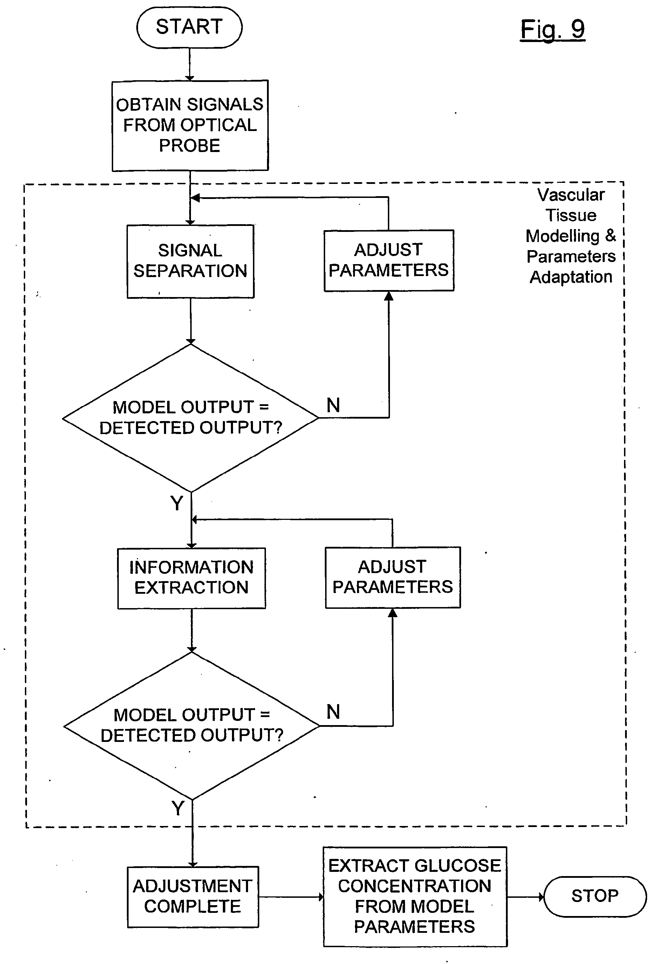

Referring now to Fig. 9, there is shown a schematic flow diagram of the processing steps accomplished by the system of this embodiment. In a first step, the processor controls the optical driver switch to illuminate each of the emitters in turn and controls the multiplexer to switch to the appropriate channel for each emitter detector pair. Once the processor has gathered data for all emitter detector pairs, processing moves to the aforementioned signal separation stage of the "vascular tissue modelling & parameters adaptation" method described in detail above, and an equation is constructed for each emitter detector pair.

The processor then compares the intensity derived from the model with that measured, and if the model intensity differs from the measured intensity by more than a predetermined amount, the processor adjusts the parameters for the constructed equations and repeats the signal separation process.

Once the model intensity output equals - to an acceptable level of accuracy - the measured intensity output, the function approximating for losses occurring due to scattering and the function approximating for losses occurring due to absorption can be determined and processing moves to the above described information extraction stage of the method.

In this stage of the method, the processor builds the subject model and tests that model to determine whether the model output is the same as (again to an acceptable level of accuracy) the measured output. If a difference is found the parameters of the model are adjusted and the model is again tested, and this process continues until the model output is substantially the same as the measured output.

Once the model has been constructed to give an output that is acceptably similar to the measured output, adjustment of the model is complete and the equations mentioned above can be solved to yield an estimation of the concentration of the substance of interest, in this example glucose, for relay to the user.

As will be appreciated from the foregoing, the system and method of the present invention provide a swift, truly non-invasive mechanism for the accurate estimation of substance concentrations in bodily fluids.

Many modifications and alterations may be made to the particular embodiments described above, and all such modifications are to be considered to be within the scope of the invention. For example, whilst the methods and techniques described herein have been implemented in software, it will be immediately apparent to persons of ordinary skill in the art that the functionality provided by that software may alternatively be provided by hardware, for example by means of an application specific integrated circuit, or indeed by a mixture of hardware and software, or indeed that the functionality provided by some of the hardware may be implemented in software. As such, the foregoing description should be read as being merely illustrative, rather than limiting the scope of the present invention.

It is also the case that whilst the embodiments disclosed above employ emitters and detectors arranged side-by-side, it will be apparent to persons skilled in the art that the emitters and detectors may instead be arranged on opposite sides of the subject so as to measure light transmitted transversely through the subject. In addition, whilst the system disclosed herein includes a computer and a separate estimation module, it will be apparent that this arrangement could be varied so that the computer and estimation module are integrated into a single device, for example into a hand-holdable device.

It should also be noted that the applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

**Claims**

1. A method for estimating the optical response of a subject comprising blood, the method comprising:

   forming a model whereby the spectral response of a subject to incident light can be estimated, the model including a plurality of parameters, each parameter corresponding to the influence on the spectral response of a respective biochemical substance;
   directing light of a known spectral content at the subject;
   detecting the spectral response of the subject to the light; and
   repeatedly performing the steps of:

   i. modelling the spectral response of the subject to the light by means of the model;
   ii. comparing the modelled spectral response with the detected response; and
   iii. altering the model in dependence on that comparison by varying one or more of the said parameters.

2. A method as claimed in claim 1, wherein the step of altering the model is only performed if the modelled spectral response is closer to the detected response than the modelled spectral response of the current model.

3. A method as claimed in any of claim 1 or 2, wherein light comprising multiple wavelengths is directed at the subject and the spectral response of the subject is detected for each respective wavelength.

4. A method as claimed in claim 3, wherein the light directed at the subject is generated by multiple devices, each being capable of generating light consisting of a single respective wavelength.

5. A method as claimed in claim 3 or 4, wherein the number of multiple wavelengths is greater than or equal to the number of parameters included in the model.

6. A method as claimed in any preceding claim, wherein the steps of modelling, comparing and altering are performed repeatedly until a difference between the modelled spectral response and the detected response is below a predetermined threshold.

7. A method as claimed in any preceding claim, wherein the step of altering the model is performed using a neural network.

8. A method as claimed in any preceding claim, wherein the spectral response of the subject is detected in light passing through at least two different paths through the subject.

9. A method as claimed in any preceding claim, wherein the model includes parameters representing the light scattering and light absorption properties of the biochemical components.

10. A method as claimed in any preceding claim, wherein the parameters include the concentrations of one or more of fat, water and glucose in the subject.

11. A method as claimed in claim 10 as dependent directly or indirectly on claim 3, wherein the multiple wavelengths include $2.10 \mu m$, $2.27 \mu m$ and $2.32 \mu m$.

12. A method for manufacturing a non-invasive sensor for estimating the concentration of a biochemical component in a subject comprising blood, the method comprising
    forming an altered model by the method of any preceding claim;
    forming a processing device configured to apply the model so as to estimate, in dependence on the spectral response of a subject to light of a known spectral content, the influence on the spectral response of the biochemical component; and
    providing output means whereby the processing device can cause data generated in dependence on the estimated influence to be output to a user.

13. A method for estimating the concentration of a.biochemical component in a subject comprising blood, the method comprising:

> forming an altered model by the method of any of claims 1 to 11;
> directing light of a known spectral content at the subject;
> detecting the spectral response of the subject to the light; and
> applying the model so as to estimate, in dependence on the spectral response of the subject to the light, the influence on the spectral response of the biochemical component; and
> in dependence on the estimated influence, estimating the concentration of the biochemical component in the subject.

14. A system (500) for estimating substance concentrations in bodily fluids, the system comprising:

> means (101) for illuminating a subject comprising bodily fluid with light having a known spectral content;
> means (102) for detecting the subject's spectral response to said light; and
> processing means (506) operable to establish a model of the subject's spectral response to irradiation with light of said known spectral content, the model having a plurality of parameters each of which corresponds to a respective biochemical substance's influence on the spectral response;
> wherein said processing means (506) is further operable to compare the modelled spectral response with the detected spectral response, and in the event that said modelled spectral response differs from said detected spectral response by more than a predetermined amount to iteratively adjust one or more of said parameters until said difference is less than said predetermined amount.

15. A system according to Claim 14, wherein said processing means is further operable, following an adjustment which yields a difference that is less than said predetermined amount, to derive from said model an estimation of the concentration of a said biochemical component in said subject.

16. A system according to Claim 14 or 15, further comprising a probe , the probe comprising a plurality of optical emitters (S$_1$, S$_2$, S$_3$) and one or more photodetectors (D1, D2) arranged side-by-side in a single housing, the arrangement being such that when the probe is placed adjacent to a subject, the plurality of optical emitters are operable to illuminate the subject with light of a known spectral content, a least a portion of which travels through the subject for detection by one or more of said photodetectors.

17. A computer program comprising one or more computer program elements that are operable, when executed in an execution environment, to form a model whereby the spectral response of a subject to incident light can be estimated, the model including a plurality of parameters, each parameter corresponding to the influence on the spectral response of a respective biochemical substance, said program being further operable to model the spectral response of the subject to light of a known spectral content directed at the subject; and to compare the modelled spectral response to a detected spectral response of the subject to the light of known spectral content.

18. A computer program according to Claim 17, wherein the computer program is operable to iteratively adapt said model in dependence upon the comparison of said modelled response to said detected response until a difference between said modelled spectral response and said detected spectral response is less than a predetermined amount.

19. A computer program according to Claim 18, wherein the computer program is operable, once said difference is less than said predetermined amount, to derive from said modelled spectral response an estimated concentration of a substance in said subject.

**Patentansprüche**

1. Verfahren zum Schätzen der optischen Reaktion eines Blut enthaltenden Subjekts, wobei
ein Modell gebildet wird, mit dem sich die spektrale Reaktion eines Subjekts auf einfallendes Licht schätzen lässt, wobei das Modell mehrere Parameter enthält, deren jeder dem Einfluss einer jeweiligen biochemischen Substanz auf die spektrale Reaktion entspricht,
Licht eines bekannten Spektralgehalts auf das Subjekt gerichtet wird,
die spektrale Reaktion des Subjekts auf das Licht gemessen wird und
die folgenden Schritte wiederholt durchgeführt werden:

i. Modellieren der spektralen Reaktion des Subjekts auf das Licht anhand des Modells,

ii. Vergleichen der modellierten spektralen Reaktion mit der gemessenen Reaktion und

iii. Ändern des Modells in Abhängigkeit von dem Vergleich durch Variieren einer oder mehrerer der Parameter.

2. Verfahren nach Anspruch 1, wobei das Ändern des Modells nur dann erfolgt, wenn die modellierte spektrale Reaktion der gemessenen Reaktion näher kommt als die modellierte spektrale Reaktion des gegenwärtigen Modells.

3. Verfahren nach Anspruch 1 oder 2, wobei Licht, das mehrere Wellenlängen enthält, auf das Subjekt gerichtet und die spektrale Reaktion des Subjekts für jede Wellenlänge gemessen wird.

4. Verfahren nach Anspruch 3, wobei das auf das Subjekt gerichtete Licht von mehreren Einrichtungen erzeugt wird, deren jede die Erzeugung von Licht einer einzigen Wellenlänge gestattet.

5. Verfahren nach Anspruch 3 oder 4, wobei die Anzahl an Wellenlängen größer oder gleich der Anzahl der in dem Modell enthaltenen Parameter ist.

6. Verfahren nach einem der vorhergehenden Ansprüche, wobei das Modellieren, Vergleichen und Ändern wiederholt so lange durchgeführt werden, bis eine Differenz zwischen der modellierten spektralen Reaktion und der gemessenen Reaktion einen vorgegebenen Schwellenwert unterschreitet.

7. Verfahren nach einem der vorhergehenden Ansprüche, wobei das Ändern des Modells unter Verwendung eines neuralen Netzes erfolgt.

8. Verfahren nach einem der vorhergehenden Ansprüche, wobei die spektrale Reaktion des Subjekts gemessen wird, indem Licht auf mindestens zwei unterschiedlichen Pfaden durch das Subjekt geleitet wird.

9. Verfahren nach einem der vorhergehenden Ansprüche, wobei das Modell Parameter enthält, die die Lichtstreu- und Lichtabsorptionseigenschaften der biochemischen Komponenten darstellen.

10. Verfahren nach einem der vorhergehenden Ansprüche, wobei zu den Parametern die Konzentrationen von Fett und/oder Wasser und/oder Glycose in dem Subjekt gehören.

11. Verfahren nach Anspruch 10, soweit dieser direkt oder indirekt auf Anspruch 3 rückbezogen ist, wobei zu den mehreren Wellenlängen 2,10 $\mu$m, 2,27 $\mu$m und 2,32 $\mu$m gehören.

12. Verfahren zum Herstellen eines nicht-invasiven Sensors zum Schätzen der Konzentration einer biochemischen Komponente in einem Blut enthaltenden Subjekt, wobei
gemäß dem Verfahren nach einem der vorhergehenden Ansprüche ein geändertes Modell gebildet wird,
eine Verarbeitungseinrichtung ausgebildet wird, die das Modell derart anzuwenden gestattet, dass sie in Abhängigkeit von der spektralen Reaktion eines Subjekts auf Licht eines bekannten Spektralgehalts den Einfluss der biochemischen Komponente auf die spektrale Reaktion schätzt, und
eine Ausgabeeinrichtung vorgesehen wird, an der das Verarbeitungseinrichtung in Abhängigkeit von dem geschätzten Einfluss erzeugte Daten an einen Benutzer ausgeben kann.

13. Verfahren zum Schätzen der Konzentration einer biochemischen Komponente in einem Blut enthaltenden Subjekt, wobei
gemäß dem Verfahren nach einem der Ansprüche 1 bis 11 ein geändertes Modell gebildet wird,
Licht eines bekannten Spektralgehalts auf das Subjekt gerichtet wird,
die spektrale Reaktion des Subjekts auf das Licht gemessen wird und
das Modell so angewendet wird, dass es in Abhängigkeit von der spektralen Reaktion des Subjekts auf das Licht den Einfluss der biochemische Komponente auf die spektrale Reaktion schätzt, und
in Abhängigkeit von dem geschätzten Einfluss die Konzentration der biochemischen Komponente in dem Subjekt schätzt.

14. System (500) zum Schätzen von Substanzkonzentrationen in Körperflüssigkeiten mit
einer Einrichtung (101) zum Bestrahlen eines eine Körperflüssigkeit enthaltenden Subjekts mit Licht eines bekannten Spektralgehalts,
einer Einrichtung (102) zum Messen der spektralen Reaktion des Subjekts auf das Licht und

einer Verarbeitungseinrichtung (506), die so arbeitet, dass sie ein Modell der spektralen Reaktion des Subjekts auf Bestrahlung mit Licht des bekannten Spektralgehalts erzeugt, wobei das Modell mehrere Parameter enthält, deren jeder dem Einfluss einer jeweiligen biochemischen Substanz auf die spektrale Reaktion entspricht,

wobei die Verarbeitungseinrichtung (506) ferner in der Lage ist, die modellierte spektrale Reaktion mit der gemessenen spektralen Reaktion zu vergleichen und, falls die modellierte spektrale Reaktion von der gemessenen spektralen Reaktion um mehr als einen vorgegebenen Betrag abweicht, einen oder mehrere der Parameter iterativ zu justieren, bis die Abweichung den vorgegebenen Betrag unterschreitet.

15. System nach Anspruch 14, wobei die Verarbeitungseinrichtung ferner in der Lage ist, nach einer Justierung, die eine Abweichung unter dem vorgegebenen Betrag ergibt, von dem Modell eine Schätzung der Konzentration der biochemischen Komponente in dem Subjekt abzuleiten.

16. System nach Anspruch 14 oder 15 mit einer Sonde, die mehrere optische Sender ($S_1$, $S_2$, $S_3$) sowie einen oder mehrere Photodetektoren (D1, D2) seitlich nebeneinander in einem gemeinsamen Gehäuse aufweist, wobei die Anordnung so getroffen ist, dass dann, wenn die Probe nahe einem Subjekt plaziert wird, die mehreren optischen Sender in der Lage sind, das Subjekt mit Licht eines bekannten Spektralgehalts zu bestrahlen, von dem mindestens ein Teil das Subjekt zur Messung mittels des bzw. der Photodetektoren durchsetzt.

17. Computerprogramm mit einem oder mehreren Computerprogramm-Elementen, die es bei Ausführung in einer Ausführungsumgebung gestatten, ein Modell zu bilden, mit dem sich die spektrale Reaktion eines Subjekts auf einfallendes Licht schätzen lässt, wobei das Modell mehrere Parameter enthält, deren jeder dem Einfluss einer betreffenden biochemischen Substanz auf die spektrale Reaktion entspricht, wobei das Programm ferner in der Lage ist, die spektrale Reaktion des mit Licht eines bekannten Spektralgehalts bestrahlten Subjekts zu modellieren und die modellierte spektrale Reaktion mit einer gemessenen spektralen Reaktion des Subjekts auf Licht des bekannten Spektralgehalts zu vergleichen.

18. Computerprogramm nach Anspruch 17, das es gestattet, das Modell in Abhängigkeit von dem Vergleich der modellierten Reaktion mit der gemessenen Reaktion iterativ so lange anzupassen, bis eine Differenz zwischen der modellierten spektralen Reaktion und der gemessenen spektralen Reaktion einen vorgegebenen Betrag unterschreitet.

19. Computerprogramm nach Anspruch 18, das es gestattet, von der modellierten spektralen Reaktion eine geschätzte Konzentration einer Substanz in dem Subjekt abzuleiten, sobald die besagte Differenz den vorgegebenen Betrag unterschreitet.

**Revendications**

1. Procédé pour évaluer la réponse optique d'un sujet comprenant du sang, le procédé comprenant :

   la formation d'un modèle grâce auquel la réponse spectrale d'un sujet à un rayon incident peut être évaluée, le modèle comprenant une pluralité de paramètres, chaque paramètre correspondant à l'influence sur la réponse spectrale d'une substance biochimique respective ;
   l'orientation de la lumière d'un contenu spectral connu sur le sujet ;
   la détection de la réponse spectral du sujet à la lumière ; et
   la réalisation répétée des étapes de :

   i. la modélisation de la réponse spectrale du sujet à la lumière au moyen du modèle ;
   ii. la comparaison de la réponse spectrale modélisée avec la réponse détectée ; et
   iii. la modification du modèle selon cette comparaison en faisant varier un ou plusieurs desdits paramètres.

2. Procédé selon la revendication 1, dans lequel l'étape de modification du modèle est seulement réalisée si la réponse spectrale modélisée est plus proche de la réponse détectée que la réponse spectral modélisée du modèle réel.

3. Procédé selon l'une quelconque des revendications 1 ou 2, dans lequel la lumière comprenant des longueurs d'ondes multiples est orientée sur le sujet et la réponse spectrale du sujet est détectée pour chaque longueur d'onde respective.

**4.** Procédé selon la revendication 3, dans lequel la lumière orientée sur le sujet est générée au moyen de dispositifs multiples, chacun étant capable de générer de la lumière consistant en une longueur d'onde respective.

**5.** Procédé selon les revendications 3 ou 4, dans lequel le nombre de longueurs d'ondes multiples est supérieur ou égal au nombre de paramètres compris dans le modèle.

**6.** Procédé selon l'une quelconque des revendications précédentes, dans lequel les étapes de modélisation, comparaison et modification sont réalisées de façon répétée jusqu'à ce qu'une différence entre la réponse spectrale modélisée et la réponse détectée soit inférieure à un seuil prédéterminé.

**7.** Procédé selon l'une quelconque des revendications précédentes, dans lequel l'étape de modification du modèle est réalisée en utilisant un réseau neural.

**8.** Procédé selon l'une quelconque des revendications précédentes, dans lequel la réponse spectrale du sujet est détectée avec une lumière passant à travers au moins deux voies différentes à travers le sujet.

**9.** Procédé selon l'une quelconque des revendications précédentes, dans lequel le modèle comprend des paramètres représentant des propriétés de diffusion de lumière et d'absorption de lumière des composants biochimiques.

**10.** Procédé selon l'une quelconque des revendications précédentes, dans lequel les paramètres comprennent des concentrations d'une ou plusieurs matières grasses, eau et glucose dans le sujet.

**11.** Procédé selon la revendication 10 dépendant directement ou indirectement de la revendication 3, dans lequel les longueurs d'ondes multiples comprennent 2,10 $\mu$m, 2,27 $\mu$m et 2,32 $\mu$m.

**12.** Procédé pour fabriquer un capteur non invasif pour évaluer la concentration d'un composant biochimique chez un sujet comprenant du sang, le procédé comprenant
la formation d'un modèle modifié au moyen du procédé selon l'une quelconque des revendications précédentes ;
la formation d'un dispositif de traitement configuré pour s'appliquer au modèle de façon à évaluer, en fonction de la réponse spectrale du sujet à une lumière d'un contenu spectral connu, l'influence de la réponse spectrale du composant biochimique ; et
fournissant un moyen de sortie grâce auquel le dispositif de traitement peut provoquer des données générées selon l'influence évaluée de la sortie à un utilisateur.

**13.** Procédé pour évaluer la concentration d'un composant biochimique chez un sujet comprenant du sang, le procédé comprenant :
la formation d'un modèle modifié selon l'une quelconque des revendications 1 à 11 ;
l'orientation de la lumière d'un contenu spectral connu vers le sujet ;
la détection de la réponse spectrale du sujet à la lumière ; et
l'application au modèle de façon à évaluer, en fonction de la réponse spectrale du sujet à la lumière, l'influence sur la réponse spectrale du composant biochimique ; et
selon l'influence évaluée, l'estimation de la concentration du composant biochimique chez le sujet.

**14.** Système (500) pour évaluer des concentrations de substance dans des fluides corporels, le système comprenant :

un moyen (110) pour éclairer un sujet comprenant un fluide corporel avec de la lumière ayant un contenu spectral connu ;
un moyen de traitement (506) fonctionnant pour établir un modèle de la réponse spectrale du sujet à l'irradiation avec la lumière dudit contenu spectral connu, le modèle ayant une pluralité de paramètres chacun desquels correspondant à l'influence d'une substance biochimique respective sur la réponse spectrale ;
dans lequel ledit moyen de traitement (506) fonctionne de plus pour comparer la réponse spectrale modélisée avec la réponse spectrale détectée, et dans l'éventualité où ladite réponse spectrale modélisée diffère de ladite réponse spectrale détectée de plus d'une quantité prédéterminée régler de façon itérative un ou plusieurs desdits paramètres jusqu'à ce que ladite différence soit inférieure à ladite quantité prédéterminée.

**15.** Procédé selon la revendication 14, dans lequel ledit moyen de traitement fonctionne de plus, en suivant un réglage qui produit une différence qui est inférieure à ladite quantité prédéterminée, pour dériver dudit modèle une évaluation de la concentration d'un dit composant biochimique dans ledit sujet.

**16.** Système selon la revendication 14 ou 15, comprenant de plus une sonde, la sonde comprenant une pluralité d'émetteurs optiques ($S_1$, $S_2$, $S_3$) et un ou plusieurs photo détecteurs ($D_1$, $D_2$) arrangés côte à côte dans un seul boîtier, l'arrangement étant tel que lorsque la sonde est placée de façon adjacente à un sujet, la pluralité d'émetteurs optiques fonctionne pour éclairer le sujet avec la lumière d'un contenu spectral connu, au moins une partie de laquelle circule à travers le sujet pour la détection au moyen d'une ou plusieurs desdits photo détecteurs.

**17.** Logiciel d'ordinateur comprenant un ou plusieurs éléments de logiciel d'ordinateur qui fonctionnent, lorsqu'ils sont exécutés dans un environnement d'exécution, pour former un modèle grâce auquel la réponse spectrale d'un sujet à la lumière incidente peut être évaluée, le modèle comprenant une pluralité de paramètres, chaque paramètre correspondant à l'influence sur la réponse spectrale d'une substance chimique respective, ledit programme fonctionnant de plus pour modéliser la réponse spectrale du sujet à la lumière d'un contenu spectral connu dirigée sur le sujet ; et pour comparer la réponse spectrale modélisée à une réponse spectrale détectée du sujet à la lumière du contenu spectral connu.

**18.** Logiciel d'ordinateur selon la revendication 17, dans lequel le logiciel d'ordinateur fonctionne pour adapter de façon itérative ledit modèle en fonction de la comparaison de ladite réponse modélisée à ladite réponse détectée jusqu'à ce qu'un différence entre ladite réponse spectrale modélisée et ladite réponse spectrale détectée soit inférieure à une quantité prédéterminée.

**19.** Logiciel d'ordinateur selon la revendication 18, dans lequel le logiciel d'ordinateur fonctionne, une fois que la différence est inférieure à ladite quantité prédéterminée, pour dériver de ladite réponse spectrale modélisée une concentration évaluée d'une substance chez ledit sujet.

## Fig. 1

## Fig. 4

EP 1 959 819 B1

## Fig. 2

$$I_{dA} = f1(x,y,z)$$
$$I_{dB} = f2(x,y,z)$$
$$I_{dC} = f3(x,y,z)$$

204

X

$I_e$

203

$I_d$

201

202

$I_e$

A  B  C  → λ

$I_d$

A  B  C  → λ

## Fig. 3

Control information set

Emited optical radiation $I_{E\lambda}$

Detected optical radiation $I_{D\lambda}$

Detected information set

System input control

Optical emitter

Vascular tissue

Optical detector

Hardware

Software

Optical emiter emulator

Vascular tissue model

Optical detector emulator

Comparator

Control information ojustment

Model of emited optical radiation

Model of detected optical radiation

Modelled information set

Physical object model porometers

Model-Detection difference

$$X = \{x_1, x_2, x_3 \dots x_n\}$$

Model porameters adaptation

## Fig. 5

## Fig. 6

Fig. 7

```
                          ┌──────────────┐
                          │     DAC      │
                          ├──────────────┤
          ┌──────────────▶│   Voltage    │
          │ ┌────────────▶│   Source     │
          │ │             └──────┬───────┘
   ┌──────┴─┴─────┐              │
   │              │       ┌──────┴───────┐
   │              │       │   V to I     │
   │   Processor  │       │  converter   │
   │              │       └──────┬───────┘
   │              │◀═════▶┌──────┴───────┐
   │              │       │   Switch     │
   └──────────────┘       └──────┬───────┘
                    ┌──────┬─────┼──────┬──────┐
                  ┌─┴─┐  ┌─┴─┐ ┌─┴─┐  ┌─┴─┐
                  │S1 │  │S2 │ │S3 │  │S1 │
                  └───┘  └───┘ └───┘  └───┘
```

Fig. 8

```
                ┌──────────────────────────────────┐
                │ ┌────────────────────────────┐    │
   ┌────────────┴─┴─┐  ┌───┐ ┌──────┐ ┌────────┴──┐ ┌──────────┐ ┌────┐
   │                │  │   │ │ S1D1 │ │           │ │          │ │ D1 │
   │                │  │   │ └──────┘ │           │ │          │ └────┘
   │                │  │   │ ┌──────┐ │           │ │          │
   │                │  │ A │ │ S1D2 │ │   De-     │ │  I to V  │
   │   Processor    │  │ D │ └──────┘ │ Multiplexer│ │ converter│
   │                │  │ C │ ┌──────┐ │           │ │          │
   │                │  │   │ │ S2D1 │ │           │ │          │ ┌────┐
   │                │  │   │ └──────┘ │           │ │          │ │ D2 │
   │                │  │   │ ┌──────┐ │           │ │          │ └────┘
   └────────────────┘  └───┘ │ S2D2 │ └───────────┘ └──────────┘
                              └──────┘
                                 └─ 514
```

**Fig. 9**

START

OBTAIN SIGNALS
FROM OPTICAL
PROBE

SIGNAL
SEPARATION

ADJUST
PARAMETERS

Vascular
Tissue
Modelling &
Parameters
Adaptation

MODEL OUTPUT =
DETECTED OUTPUT?

N

Y

INFORMATION
EXTRACTION

ADJUST
PARAMETERS

MODEL OUTPUT =
DETECTED OUTPUT?

N

Y

ADJUSTMENT
COMPLETE

EXTRACT GLUCOSE
CONCENTRATION
FROM MODEL
PARAMETERS

STOP

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 022413 A